(19) Europäisches Patentamt / European Patent Office / Office européen des brevets

(11) **EP 3 006 961 B1**

(12) **EUROPEAN PATENT SPECIFICATION**

(45) Date of publication and mention
of the grant of the patent:
**27.02.2019 Bulletin 2019/09**

(21) Application number: **14803801.1**

(22) Date of filing: **14.01.2014**

(51) Int Cl.:
*G01T 3/00* (2006.01)      *G01T 3/06* (2006.01)
*A61N 5/10* (2006.01)

(86) International application number:
**PCT/JP2014/050457**

(87) International publication number:
**WO 2014/192321 (04.12.2014 Gazette 2014/49)**

(54) **NEUTRON RADIATION DETECTION DEVICE AND NEUTRON CAPTURE THERAPY DEVICE**

VORRICHTUNG ZUR ERKENNUNG EINER NEUTRONENSTRAHLUNG UND
NEUTRONENERFASSENDE THERAPIEVORRICHTUNG

DISPOSITIF DE DÉTECTION DE RAYONNEMENT DE NEUTRONS ET DISPOSITIF DE
TRAITEMENT PAR CAPTURE DE NEUTRONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.05.2013 JP 2013110962**

(43) Date of publication of application:
**13.04.2016 Bulletin 2016/15**

(73) Proprietor: **Sumitomo Heavy Industries, Ltd.
Tokyo 141-6025 (JP)**

(72) Inventor: **TAKI Kazuya
Yokosuka-shi
Kanagawa 237-8555 (JP)**

(74) Representative: **Louis Pöhlau Lohrentz
Patentanwälte
Postfach 30 55
90014 Nürnberg (DE)**

(56) References cited:
**JP-A- H10 160 845      JP-A- 2008 026 195
JP-A- 2013 167 467      US-A1- 2009 014 662**

- **Y. YAMANE ET AL.: 'Measurement of the thermal
and fast neutron flux in a research reactor with a
Li and Th loaded optical fibre detector' NUCLEAR
INSTRUMENTS AND METHODS IN PHYSICS
RESEARCH A vol. 432, 1999, pages 403 - 409,
XP055295146**

**Description**

Technical Field

The present invention relates to a neutron beam detection apparatus and a neutron capture therapy device including an optical fiber through which light generated in a scintillator is transmitted.

Background Art

Japanese Unexamined Patent Application Publication No. 61-71381 discloses a technology of discriminating a neutron beam from a gamma ray. When a neutron beam or a gamma ray is incident on a scintillator, scintillation light is emitted. The light generated in the scintillator is converted into an electrical pulse by a photo-multiplier. A neutron beam and a gamma ray are different from each other in electrical pulse due to a difference in deceleration times of emission of light. According to PTL 1, a neutron beam and a gamma ray are discriminated from each other based on a pulse width.

United States Patent Application US 2009/0014662 A1 discloses a neutron detector that comprises thin plastic scintillation fibers which are optically coupled via lenses to a CMOS photo-sensor array. The CMOS photo-diode outputs are amplified and fed to a discriminator, rejecting signals that are less than a predetermined level.

Summary of Invention

Technical Problem

It is conceivable that light generated in a scintillator is transmitted through an optical fiber. However, light transmittance of the optical fiber is degraded in accordance with deterioration thereof caused by radial rays. Therefore, for example, there is concern that a signal generated by a neutron beam may be erroneously detected as a signal which is generated by a gamma ray, resulting in degradation of detection accuracy of a neutron beam. An objective of an aspect of the present invention is to provide a neutron beam detection apparatus and a neutron capture therapy device in which detection accuracy of a neutron beam can be prevented from being degraded.

Solution to Problem

According to an aspect of the invention, there is provided a neutron beam detection apparatus which detects a neutron beam, the apparatus including: a scintillator configured to generate light when a radial ray is incident thereon; an optical fiber configured to cause the light generated in the scintillator to be transmitted therethrough; and a discrimination section configured to receive the light transmitted through the optical fiber and to discriminate a detection signal generated by a gamma ray from a signal related to the neutron beam when a wave height of the detection signal related to the received light exceeds a determination threshold value, wherein the discrimination section is adapted to adjust the determination threshold value in accordance with deterioration of the optical fiber.

In the neutron beam detection apparatus, light is emitted from the scintillator on which a radial ray is incident, and the emitted light is transmitted through the optical fiber and is introduced into the discrimination section. The discrimination section discriminates the detection signal from the signal related to the neutron beam when the wave height of the detection signal related to the received light exceeds the determination threshold value. Since the discrimination section of the neutron beam detection apparatus can adjust the determination threshold value in accordance with deterioration of the optical fiber, even when the optical fiber is deteriorated due to radial rays and light transmittance thereof is degraded, it is possible to prevent detection accuracy of a neutron beam from being degraded by adjusting the determination threshold value.

The discrimination section may adjust the determination threshold value based on the distribution of the wave heights of the detection signals. In this case, since the determination threshold value can be adjusted based on the distribution of the wave heights of the detection signals, it is possible to prevent detection accuracy of a neutron beam from being degraded.

The discrimination section may detect a peak generated by the neutron beam in the distribution of the wave heights and may adjust the determination threshold value based on the peak. As the optical fiber is deteriorated, the peak generated by the neutron beam is also lowered in accordance with degradation of the transmittance thereof. Since the determination threshold value is adjusted based on the peak of the neutron beam, it is possible to accurately detect the neutron beam and to prevent detection accuracy from being degraded. For example, the peak of the neutron beam in the distribution of the wave heights may be set based on past data.

The discrimination section may set a value lowered by a predetermined value from the peak as the determination threshold value. In this manner, since the value lowered by the predetermined value from the peak of the neutron beam in the distribution of the wave heights is set to the determination threshold value, it is possible to adjust the determination threshold value in accordance with deterioration of the optical fiber. For example, the predetermined value may be set based on past data.

The discrimination section may detect the peak generated by the neutron beam by fitting a Gaussian function to the distribution of the wave heights, and may cause a value lowered by 1.5 $\sigma$ from the peak to be the determination threshold value. The neutron beam detection apparatus uses the Gaussian function to perform fitting with respect to the distribution of the wave heights, thereby detecting the peak generated by the neutron beam. Since the discrimination section adjusts the determination threshold value to the value lowered by 1.5 $\sigma$ from the peak generated by the neutron beam, it is possible to prevent detection accuracy of a neutron beam further from being degraded.

The neutron beam detection apparatus may also include a light generation section. The discrimination section may adjust the determination threshold value based on an optical signal from the light generation section. Since the discrimination section adjusts the determination threshold value based on the optical signal from the light generation section, it is possible to prevent detection accuracy of a neutron beam further from being degraded, based on the optical signal from the light generation section.

The discrimination section may store the predetermined optical signal from the light generation section, may compare the stored predetermined optical signal with the actually measured optical signal which is detected via the optical fiber, and may adjust the determination threshold value based on a comparison result. Since the discrimination section adjusts the determination threshold value based on the stored predetermined optical signal and the measured optical signal, it is possible to adjust the determination threshold value in real time. Accordingly, even though the optical fiber is deteriorated, the determination threshold value can be adjusted in real time.

The light generation section may be installed at an end portion of the optical fiber on a side on which light generated in the scintillator is incident. Accordingly, it is possible to adjust the determination threshold value based on the optical signal from the light generation section which is installed at the end portion of the optical fiber.

The neutron beam detection apparatus may also include a determination section configured to determine which occurs between an occurrence of deterioration of the optical fiber caused by the radial ray and an occurrence of a problem in the discrimination section, based on the optical signal from the light generation section. Accordingly, it is possible to determine which occurs between deterioration of the optical fiber and a problem in the discrimination section, by using the optical signal from the light generation section.

The neutron beam detection apparatus may also include a light generation section optical fiber configured to transmit the optical signal generated in the light generation section. The discrimination section may also receive the optical signal transmitted through the light generation section optical fiber. The determination section may determine that there is an occurrence of a problem in the discrimination section when the wave height of the detection signal related to the light which is received by the discrimination section and is generated in the scintillator and the wave height of the detection signal related to the optical signal which is output from the light generation section are generally shifted in an increase direction or a decrease direction of the wave height, and may determine that there is an occurrence of deterioration of the optical fiber caused by the radial ray when only the wave height of the detection signal related to the light which is generated in the scintillator is shifted in the decrease direction of the wave height. In this case, the determination section can determine the occurrence of a problem in the discrimination section and deterioration of the optical fiber based on the wave height of the detection signal related to light which is generated in the scintillator and the wave height of the detection signal related to the optical signal which is output from the light generation section.

According to another aspect of the present invention, there is provided a neutron capture therapy device including the neutron beam detection apparatus.

In the neutron capture therapy device, the neutron beam detection apparatus is included, light is emitted from the scintillator on which a gamma ray or a neutron beam is incident, and the emitted light is transmitted through the optical fiber and is introduced into the discrimination section. The discrimination section discriminates the detection signal from the signal related to the neutron beam when the wave height of the detection signal related to the received light exceeds the determination threshold value. The discrimination section discriminates the detection signal from the signal related to the gamma ray when the wave height of the detection signal does not exceed the determination threshold value. Since the discrimination section of the neutron beam detection apparatus can adjust the determination threshold value in accordance with deterioration of the optical fiber, even when the optical fiber is deteriorated due to radial rays and light transmittance thereof is degraded, it is possible to prevent detection accuracy of a neutron beam from being degraded by adjusting the determination threshold value.

Advantageous Effects of Invention

According to various aspects of the present invention, it is possible to provide a neutron beam detection apparatus and a neutron capture therapy device in which detection accuracy of a neutron beam can be prevented from being degraded even though an optical fiber is deteriorated and light transmittance thereof is degraded.

Brief Description of Drawings

FIG. 1 is a schematic diagram illustrating a neutron capture therapy device which includes a neutron beam detector according to a first embodiment.
FIG. 2 is a cross-sectional diagram illustrating the neutron beam detector which is provided in a collimator.
FIG. 3 is a block diagram illustrating a control unit of the neutron capture therapy device according to the first embodiment.
FIG. 4 is a graph illustrating distribution of wave heights of detection signals.
FIG. 5 is a table illustrating a relationship between a determination threshold value $Q_{th}$ and detection efficiency of a neutron beam N.
FIG. 6 is a graph illustrating a relationship between the distribution of the wave heights of the detection signals and the determination threshold value $Q_{th}$.
FIG. 7 is a schematic diagram illustrating a light guide according to a second embodiment.
FIG. 8 is a graph illustrating the distribution of the wave heights of the detection signals.
FIG. 9 is a block diagram illustrating the control unit of the neutron capture therapy device according to the second embodiment.
FIG. 10 is a schematic diagram illustrating the neutron beam detector according to a third embodiment.
FIG. 11 is a block diagram illustrating the control unit of the neutron capture therapy device according to the third embodiment.
FIG. 12(a) is a graph illustrating the distribution of the wave heights of the detection signals in a case where a problem occurs in an optical detector, and FIG. 12(b) is a graph illustrating the distribution of the wave heights of the detection signals when deterioration occurs in the light guide.

Description of Embodiments

Hereinafter, embodiments of the present invention will be described in detail with reference to the drawings.

(First Embodiment)

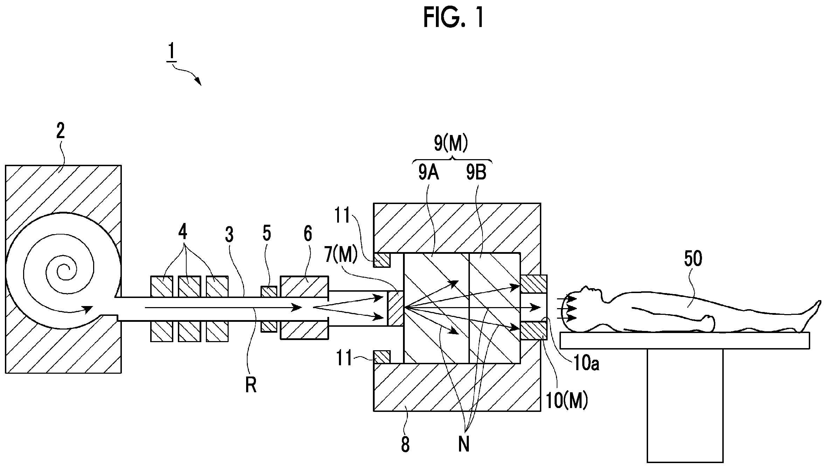

First, a first embodiment will be described. A neutron capture therapy device 1 illustrated in FIG. 1 is an apparatus for performing cancer treatment using a boron neutron capture therapy (BNCT). In the neutron capture therapy device 1, irradiation of a neutron beam N is performed with respect to a tumor of a patient (an irradiation target) 50 to whom boron ($^{10}$B) is applied, for example.
The neutron capture therapy device 1 includes a cyclotron 2. The cyclotron 2 is an accelerator which generates a charged particle beam R by accelerating a charged particle such as an anion. According to the present embodiment, the charged particle beam R is a proton beam which is generated by stripping an electrical charge from an anion. The proton beam is generated inside the cyclotron 2 by stripping an electron from an accelerated anion using a foil stripper and the like, thereby being emitted from the cyclotron 2. For example, the cyclotron 2 is capable of generating the charged particle beam R of 60 kW (= 30 MeV X 2 mA) having the beam radius of 40 mm. The accelerator may be a synchrotron, a synchrocyclotron, or a linear accelerator without being limited to the cyclotron.
The charged particle beam R emitted from the cyclotron 2 is sent to a neutron beam generation section M. The neutron beam generation section M is configured to include a target 7, a moderator 9, and a collimator 10. The charged particle beam R emitted from the cyclotron 2 passes through a beam duct 3 and travels toward the target 7 which is disposed at an end portion of the beam duct 3. A plurality of quadrupole electromagnets 4, a current detection unit 5, and a scanning electromagnet 6 are provided along the beam duct 3. The plurality of quadrupole electromagnets 4 perform beam axis adjustment or beam diameter adjustment of the charged particle beam R by using the electromagnets, for example.
The current detection unit 5 detects the current value (that is, an electrical charge, and a radiation dose rate of irradiation) of the charged particle beam R with which the target 7 is irradiated, in real time during the irradiation of the charged particle beam R. A non-destructive DC current transformer (DCCT) capable of measuring a current without affecting the charged particle beam R is used as the current detection unit 5. The current detection unit 5 outputs a detection result to a control unit 20 described below. The term "radiation dose rate" denotes a radiation dose per unit time.
Specifically, in order to accurately detect the current value of the charged particle beam R with which the target 7 is irradiated, the current detection unit 5 is provided immediately in front of the scanning electromagnet 6 on a downstream side from the quadrupole electromagnets 4 (a downstream side of the charged particle beam R) so as to eliminate the influence caused by the quadrupole electromagnets 4. In other words, since the scanning electromagnet 6 performs scanning so as to not irradiate the same place of the target 7 with the charged particle beam R at all times, in a case where the current detection unit 5 is arranged on the downstream side from the scanning electromagnet 6, a large-sized current detection unit 5 is necessary. In contrast, the current detection unit 5 can be reduced in size by providing the current detection unit 5 on an upstream side from the scanning electromagnet 6.

The scanning electromagnet 6 scans the charged particle beam R, thereby controlling irradiation of the charged particle beam R with respect to the target 7. The scanning electromagnet 6 controls an irradiation position of the charged particle beam R with respect to the target 7.

The neutron capture therapy device 1 generates the neutron beam N by irradiating the target 7 with the charged particle beam R, thereby emitting the neutron beam N toward the patient 50. The neutron capture therapy device 1 includes the target 7, a beam blockage body 8, the moderator 9, the collimator 10, and a gamma ray detection unit 11.

In addition, the neutron capture therapy device 1 includes the control unit 20. The control unit 20 is configured to include a central processing unit [CPU], a read-only memory [ROM], a random access memory [RAM], and the like. The control unit 20 is an electronic control unit which generally controls the neutron capture therapy device 1.

The target 7 generates the neutron beam N upon the reception of irradiation which is performed with the charged particle beam R. In this case, the target 7 is formed of beryllium (Be), lithium (Li), tantalum (Ta), or tungsten (W), for example, and exhibits a circular plate shape having a diameter of 160 mm. The target 7 may be in a liquid state, for example, without being limited to the plate shape.

The moderator 9 moderates energy of the neutron beam N generated in the target 7. The moderator 9 has a layered structure configured to include a first moderator 9A which mainly moderates fast neutrons included in the neutron beam N, and a second moderator 9B which mainly moderates epithermal neutrons included in the neutron beam N.

The beam blockage body 8 blocks the generated neutron beam N, a gamma ray which is generated in response to the generation of the neutron beam N, and the like so as not to allow the beam and the ray to be radiated to the outside. The beam blockage body 8 is provided so as to surround the moderator 9. An upper portion and a lower portion of the beam blockage body 8 extend from the moderator 9 to the upstream side of the charged particle beam R, and the gamma ray detection unit 11 is provided in each of the extension portions thereof.

The collimator 10 performs shaping of the irradiation field of the neutron beam N and has an opening 10a through which the neutron beam N passes. For example, the collimator 10 is a block-shaped member having the opening 10a at the center.

The gamma ray detection unit 11 detects a gamma ray generated from the neutron beam generation section M in response to the irradiation of the charged particle beam R in real time while generating a neutron beam (in other words, while irradiating the patient 50 with the neutron beam N). As the gamma ray detection unit 11, it is possible to employ various gamma ray detection instruments such as a scintillator, an ionization chamber, and others. In the present embodiment, the gamma ray detection unit 11 is provided on the upstream side of the charged particle beam R from the moderator 9 on the periphery of the target 7.

The gamma ray detection unit 11 is disposed on each of the inner sides of the upper portion and the lower portion of the beam blockage body 8 which extends to the upstream side of the charged particle beam R. The number of gamma ray detection units 11 may be one or may be three or more, without being particularly limited. When providing three or more gamma ray detection units 11, the gamma ray detection units 11 can be provided at predetermined intervals so as to surround the outer circumference of the target 7. The gamma ray detection unit 11 outputs a detection result of a gamma ray to the control unit 20. The configuration including no gamma ray detection unit 11 may be adopted.

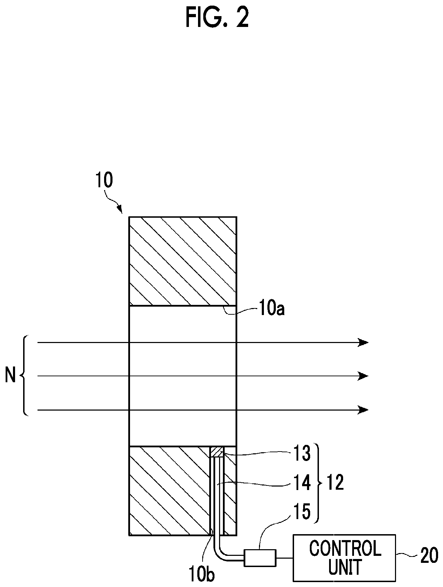

As illustrated in FIG. 2, the collimator 10 is provided with a neutron beam detector 12 for detecting the neutron beam N which passes through the opening 10a of the collimator 10 in real time while generating a neutron beam (in other words, while irradiating the patient 50 with the neutron beam N) . At least a portion of the neutron beam detector 12 is provided in a penetration hole 10b (a penetration hole formed in a direction orthogonal to the opening 10a) formed in the collimator 10. The neutron beam detector 12 includes a scintillator 13, a light guide 14, and an optical detector 15.

The scintillator 13 is a phosphor which converts an incident radial ray (the neutron beam N or the gamma ray) into light. The internal crystal of the scintillator 13 is in an excitation state in accordance with the radiation dose of the incident radial ray, thereby generating scintillation light. The scintillator 13 is provided inside the penetration hole 10b of the collimator 10 and is exposed to the opening 10a of the collimator 10. The scintillator 13 emits light as the neutron beam N or a gamma ray inside the opening 10a is incident on the scintillator 13. As the scintillator 13, it is possible to employ a $^6$Li glass scintillator, a LiCAF scintillator, a plastic scintillator coated with $^6$LiF, a $^6$LiF/ZnS scintillator, and the like.

In FIG. 2, the forward direction of the neutron beam N is illustrated in a straight line. However, the neutron beam N actually travels in a diffusive manner. Therefore, the scintillator 13 provided inside the penetration hole 10b is also irradiated with the neutron beam N, and thus, the neutron beam N can be detected by the scintillator 13.

The light guide 14 is a member which transfers light generated in the scintillator 13. The light guide 14 is configured to be formed with optical fibers. For example, the light guide 14 may be configured to be formed with a flexible optical fiber bundle and the like. The optical detector 15 detects light which is transferred through the light guide 14. As the optical detector 15, for example, it is possible to employ various optical detection instruments such as a photomultiplier and a phototube. The optical detector 15 outputs an electrical signal (a detection signal) to the control unit 20 when light is detected.

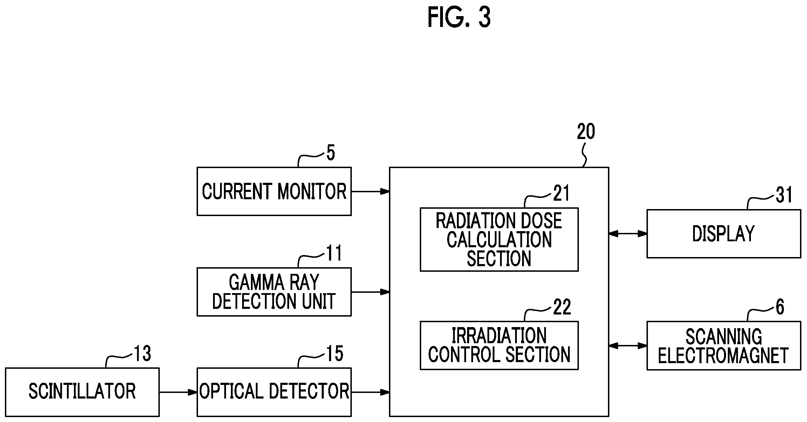

As illustrated in FIG. 3, the control unit 20 includes a radiation dose calculation section 21 and an irradiation control section 22. The control unit 20 is electrically connected to the current detection unit 5, the gamma ray detection unit 11, and the optical detector 15 (the neutron beam detector 12).

The radiation dose calculation section 21 measures (calculates) the radiation dose of the charged particle beam R with which the target 7 is irradiated, in real time during the irradiation of the charged particle beam R based on the detection result of the current value of the charged particle beam R obtained by the current detection unit 5. The radiation dose calculation section 21 performs iterated integration regarding time with the measured current value of the charged particle beam R, thereby calculating the radiation dose of the charged particle beam R in real time.

In addition, the radiation dose calculation section 21 measures (calculates) the radiation dose of a gamma ray in real time during the irradiation of the neutron beam N based on the detection result of the gamma ray obtained by the gamma ray detection unit 11.

Moreover, the radiation dose calculation section 21 measures (calculates) the radiation dose of the neutron beam N passing through the opening 10a of the collimator 10, based on the detection result of the neutron beam N obtained by the neutron beam detector 12. The radiation dose calculation section 21 receives a detection signal from the optical detector 15 and discriminates a signal related to a neutron beam from a signal related to a gamma ray (will be described later in detail). The radiation dose calculation section 21 configures a discrimination section in association with an optical detection unit 15.

The radiation dose calculation section 21 generally calculates the radiation dose of the neutron beam N generated in the target 7, in real time during the irradiation of the neutron beam N based on the radiation dose of the charged particle beam R, the radiation dose of the gamma ray, and the radiation dose of the neutron beam N which are calculated. For example, a display (a display unit) 31 displays the calculation result such as the radiation dose of the neutron beam N obtained by the radiation dose calculation section 21.

The irradiation control section 22 controls the irradiation of the charged particle beam R with respect to the target 7 based on the radiation dose of the neutron beam N which is calculated by the radiation dose calculation section 21. The irradiation control section 22 controls the irradiation of the charged particle beam R with respect to the target 7 by transmitting a command signal to the cyclotron 2 and the scanning electromagnet 6, thereby controlling the irradiation of the neutron beam N generated from the target 7, with respect to a patient. The irradiation control section 22 controls the irradiation of the neutron beam N so as to cause the radiation dose of the neutron beam N calculated by the radiation dose calculation section 21 to comply with the treatment plan which is set in advance.

The radiation dose calculation section 21 determines whether or not the wave height of the detection signal (the quantity of light) related to light received by the optical detector 15 exceeds a determination threshold value $Q_{th}$, thereby discriminating the detection signal generated by the neutron beam N and the detection signal generated by a gamma ray from each other. The neutron beam N and the gamma ray are incident on the scintillator 13 as radial rays. Therefore, the neutron beam N and a gamma ray are discriminated from each other in accordance with the intensity of the quantity of light.

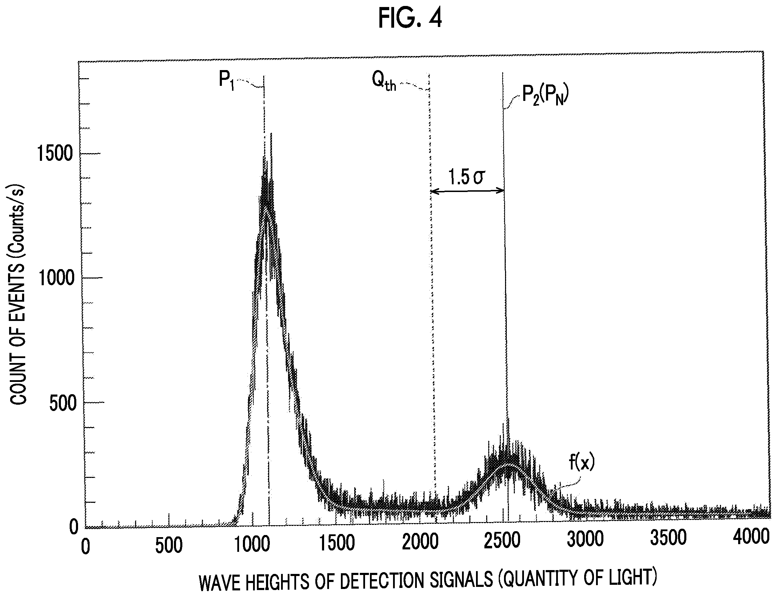

FIG. 4 is a graph illustrating distribution of the wave heights of the detection signals. In FIG. 4, the horizontal axis represents the wave height of the detection signal (the quantity of light) related to light received by the optical detector 15, and the vertical axis represents the count of events (Count/s) of the detection signals. The radiation dose calculation section 21 performs fitting with respect to the distribution of the wave heights, thereby detecting the peak in the distribution of the wave heights. As the function for the fitting, a Gaussian function in the following Expression (1) can be exemplified.

[Expression 1]

$$f(x) = \exp\left\{ -\frac{(x-\mu)^2}{2\sigma^2} \right\} \cdots (1)$$

The mark x represents a quantity of light of the detection signal, and $\mu$ represents a position of the peak.

As illustrated in FIG. 4, the radiation dose calculation section 21 sets a peak $P_2$ which is the second peak in the distribution of the wave heights as a peak $P_N$ generated by the neutron beam N and sets a value lowered by 1.5 $\sigma$ from the peak $P_N$ generated by the neutron beam N as the determination threshold value $Q_{th}$.

Here, the radiation dose calculation section 21 changes (adjusts) the determination threshold value $Q_{th}$ every

predetermined interval time period. The radiation dose calculation section 21 sets "60 seconds" as the interval time period for changing the determination threshold value $Q_{th}$, and the distribution of the wave heights is made based on the wave height of the detection signal related to the light which is received within the interval time period, thereby changing the determination threshold value $Q_{th}$ based on the distribution of the wave heights . Moreover, the interval time period may be set to a different time period without being limited to 60 seconds. The interval time period may be appropriately decided by a worker

(an operator or a doctor) . The distribution of the wave heights stands for a graph indicating the quantity of light on the horizontal axis, and indicating the frequency (the count of events) at which the detection signal of the quantity of light is detected on the vertical axis.

As the neutron capture therapy device 1 is used, the optical fiber of the light guide 14 is deteriorated due to the received radial rays, and thus, light transmittance thereof is degraded. If the light transmittance of the light guide 14 is degraded, the quantity of light measured by the radiation dose calculation section 21 decreases, and thus, the distribution of the wave heights illustrated in FIG. 4 is shifted to the left in accordance with the degradation of the transmittance. The radiation dose detection section 21 performs fitting of the Gaussian function to the distribution of the wave heights at the interval time period and sets the peak $P_N$ generated by the neutron beam N, thereby setting the value lowered by 1.5 $\sigma$ from the peak $P_N$ to a new determination threshold value $Q_{th}$. The radiation dose detection section 21 discriminates the detection signal generated by the neutron beam N and the detection signal generated by a gamma ray from each other based on the determination threshold value $Q_{th}$.

Subsequently, an operational effect of the neutron capture therapy device 1 according to the aforementioned first embodiment will be described.

In the neutron beam detector 12 of the neutron capture therapy device 1, if a gamma ray or the neutron beam N is incident thereon, the scintillator 13 emits light. The emitted light is transmitted by the light guide 14 and is introduced into the optical detector 15. The optical detection unit 15 outputs an electrical signal (a detection signal) to the control unit 20 in response to the introduced light. Since the radiation dose calculation section 21 of the control unit 20 can adjust the determination threshold value $Q_{th}$ based on the distribution of the wave heights of the detection signals, even when the optical fiber of the light guide 14 is deteriorated due to radial rays and the light transmittance is degraded, it is possible to prevent detection accuracy of the neutron beam N from being degraded, by adjusting the determination threshold value $Q_{th}$. Accordingly, the neutron beam N can be accurately detected.

In a neutron beam detection apparatus of the neutron capture therapy device 1, since the peak $P_N$ generated by a neutron beam is detected by fitting the Gaussian function to the distribution of the wave heights and the value lowered by 1.5 $\sigma$ from the peak $P_N$ is adjusted as the determination threshold value $Q_{th}$, the determination threshold value $Q_{th}$ is changed in accordance with deterioration of the optical fiber, and thus, the neutron beam N and a gamma ray can be accurately discriminated from each other. Since the peak PN is lowered in accordance with a decrease of the quantity of light, the neutron beam N and a gamma ray can be accurately discriminated from each other by changing the determination threshold value $Q_{th}$ in accordance with the peak PN.

In the neutron capture therapy device 1, the radiation dose of the neutron beam N can be precisely measured, and thus, it is possible to confirm that irradiation of the neutron beam N is performed as planned.

The present invention is not limited to the above-described first embodiment, and various changes can be made as follows without departing from the scope of the concept according to the present invention.

For example, the place for the scintillator 13 to be disposed may be other places without being limited to the inside of the collimator 10. For example, the scintillator may be disposed on the downstream side of the collimator 10 or may be disposed on a surface (in the vicinity of an irradiated portion) of a patient.

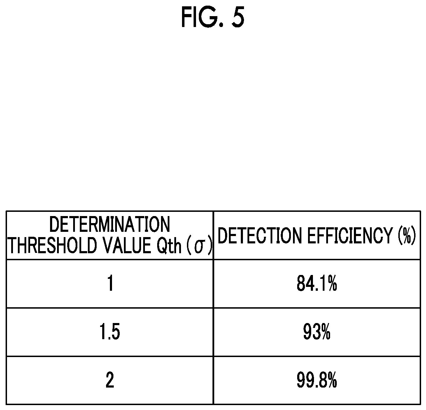

In addition, according to the above-described first embodiment, the value lowered by 1.5 $\sigma$ from the peak $P_N$ generated by the neutron beam N is adjusted as the determination threshold value $Q_{th}$. However, the determination threshold value $Q_{th}$ may be changed so as to obtain a different value. FIG. 5 is a table illustrating a relationship between the determination threshold value $Q_{th}$ and detection efficiency of the neutron beam N. As illustrated in FIG. 5, when a value lowered by $\sigma$ from the peak $P_N$ generated by the neutron beam N is caused to be the determination threshold value $Q_{th}$, the detection efficiency of the neutron beam N is 84.1%. When the value lowered by 1.5 $\sigma$ from the peak $P_N$ generated by the neutron beam N is caused to be the determination threshold value $Q_{th}$, the detection efficiency of the neutron beam N is 93%. In addition, when a value lowered by 2 $\sigma$ from the peak $P_N$ generated by the neutron beam N is caused to be the determination threshold value $Q_{th}$, the detection efficiency of the neutron beam N is 99.8%. In this manner, the value lowered by $\sigma$ from the peak $P_N$ generated by the neutron beam N may be the determination threshold value $Q_{th}$, the value lowered by 2 $\sigma$ from the peak $P_N$ generated by the neutron beam N may be the determination threshold value $Q_{th}$, and other lowered values may be the determination threshold value $Q_{th}$.

In addition, according to the above-described first embodiment, the peak $P_N$ generated by a neutron beam is detected by fitting the Gaussian function to the distribution of the wave heights. However, the peak $P_N$ generated by a neutron beam may be detected by approximating the distribution of the wave heights using other functions.

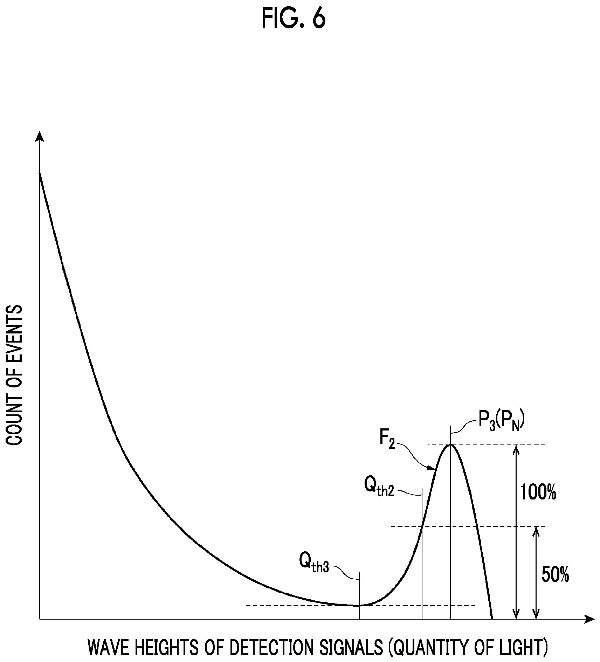

In addition, other values may be used as the determination threshold value $Q_{th}$. FIG. 6 is a graph illustrating a relationship between the distribution of the wave heights of the detection signals and the determination threshold value $Q_{th}$. As illustrated in FIG. 6, for example, when the count of events for the peak $P_N$ generated by a neutron beam is set to be 100%, a value at which the count of events becomes 50% (a value on the low side in the Gaussian distribution) may be employed as the determination threshold value $Q_{th}$ ($Q_{th2}$). In addition, a value corresponding to the peak $P_N$ ($P_3$) may be employed as the determination threshold value $Q_{th}$, and a value lowered by a predetermined value from the peak $P_N$ may be employed as the determination threshold value $Q_{th}$. The predetermined value may be set based on past data.

In addition, the determination threshold value $Q_{th}$ may be adjusted based on the inclination of the Gaussian function corresponding to the distribution of the wave heights. For example, a point at which the inclination of the Gaussian function becomes zero as illustrated in FIG. 6 may be employed as the determination threshold value $Q_{th}$ ($Q_{th3}$).

(Second Embodiment)

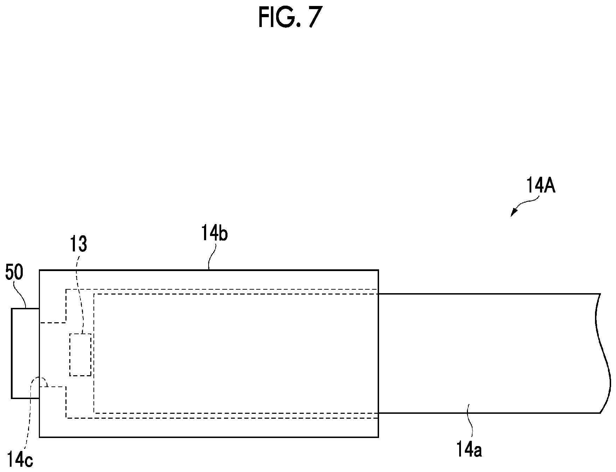

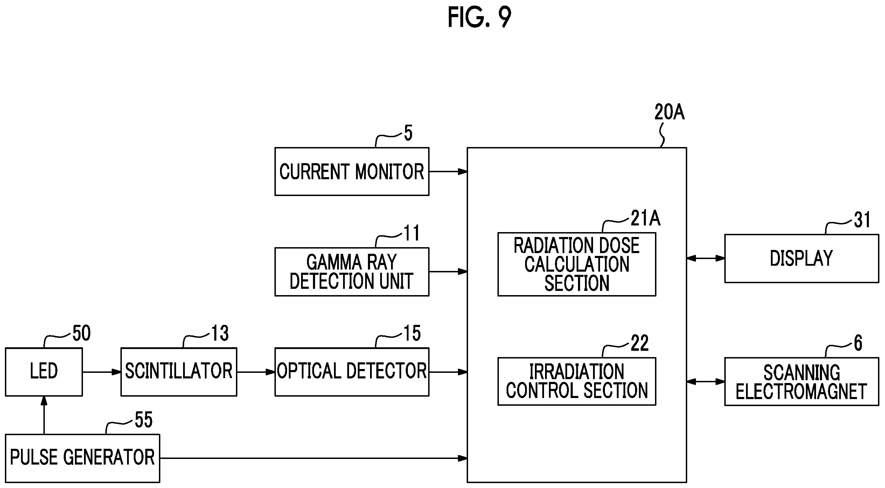

Subsequently, a second embodiment will be described. The main point of the present embodiment is that a light guide 14A (refer to FIG. 7) having a different configuration is used in place of the light guide 14 in the first embodiment. In addition, the present embodiment uses a control unit 20A (refer to FIG. 9) of which the processing contents are different from that of the control unit 20 in the first embodiment. Hereinafter, descriptions will be given mainly regarding the points different from those in the first embodiment. In addition, the same reference numerals and signs are applied to the same elements as those in the first embodiment in the descriptions for the drawings, and overlapping descriptions will be omitted.

As illustrated in FIG. 7, similar to the light guide 14 in the first embodiment, the light guide 14A used in the present embodiment transmits light generated in the scintillator 13 to the optical detector 15. The light guide 14 is configured to include an optical fiber portion 14a for transmitting light, and a cap 14b for covering an end portion of the optical fiber portion 14a.

The cap 14b covers the end portion of the optical fiber portion 14a together with the scintillator 13. An LED (a light emitting diode, a light generation section) 50 is installed at a tip end portion of the cap 14b. An opening portion 14c is provided at a portion in the cap 14b facing the end portion of the optical fiber portion 14a.

Emission of light in the LED 50 is controlled by a pulse generator 55 (refer to FIG. 9) . The LED 50 is controlled by the pulse generator 55 and outputs a pulse-like optical signal. The optical signal generated in the LED 50 is incident on the optical fiber portion 14a via the opening portion 14c of the cap 14b. The optical signal incident on the optical fiber portion 14a is transmitted to the optical detector 15 by the optical fiber portion 14a.

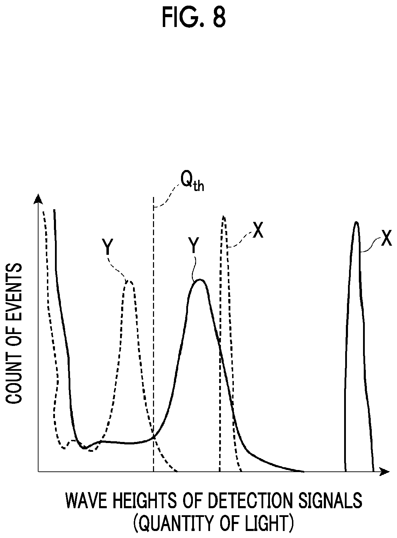

FIG. 8 illustrates a graph of the distribution of the wave heights of the detection signals related to light received by the optical detector 15. As illustrated in FIG. 8, the graph of the distribution of the wave heights includes distribution of the wave heights X of the detection signals related to the optical signal generated in the LED 50, and distribution of the wave heights Y of the detection signals related to light generated in the scintillator 13.

As illustrated in FIG. 9, the control unit 20A used in the present embodiment includes a radiation dose calculation section 21A and the irradiation control section 22. The radiation dose calculation section 21A determines whether or not the wave height of the detection signal (the quantity of light) related to light received by the optical detector 15 exceeds the determination threshold value $Q_{th}$, thereby discriminating the detection signal generated by the neutron beam N and the detection signal generated by a gamma ray from each other.

In addition, the radiation dose calculation section 21A stores the distribution of the wave heights of the detection signals related to the optical signal from the LED 50 in advance. Since the LED 50 is a stable light source, the LED 50 exhibits constant distribution of the wave heights unless the light guide 14A is deteriorated. As an example, the radiation dose calculation section 21A stores the distribution of the wave heights of the detection signals related to the optical signal from the LED 50 in advance in a case where the light guide 14A is not deteriorated.

Subsequently, descriptions will be given regarding processing in which the determination threshold value $Q_{th}$ is changed when the radiation dose calculation section 21A discriminates the detection signal generated by the neutron beam N and the detection signal generated by a gamma ray from each other. The radiation dose calculation section 21A compares the distribution of the wave heights of the detection signals stored in advance and related to the optical signal from the LED 50, and the distribution of the wave heights of the detection signals actually measured by the optical detector 15 and related to the optical signal from the LED 50.

Here, as illustrated in FIG. 8, as the light guide 14A is deteriorated, the wave heights are shifted in the decrease direction in the graph of the distribution of the wave heights. FIG. 8 illustrates the distribution of the wave heights after being shifted due to deterioration of the light guide 14A, by using a dotted line.

Therefore, the radiation dose calculation section 21A can determine whether or not there is an occurrence of deterioration of the light guide 14 by comparing the distribution of the wave heights of the detection signals stored in advance and related to the optical signal from the LED 50, and the distribution of the wave heights of the detection signals actually measured by the optical detector 15 and related to the optical signal from the LED 50.

When there is an occurrence of deviation in two cases of the distribution of the wave heights, the radiation dose calculation section 21A changes the determination threshold value $Q_{th}$. For example, the radiation dose calculation section 21A can change the determination threshold value $Q_{th}$ to a value corresponding to the quantity of light (as an example, the quantity of light of 45%) of the LED 50 detected by the optical detecting unit 15. It is possible to set the determination threshold value $Q_{th}$ to an optimal value without analyzing the distribution of neutrons, by changing the determination threshold value $Q_{th}$ based on the quantity of light of the LED 50. In this manner, the radiation dose calculation section 21A configures the discrimination section in association with the optical detection unit 15.

In addition, the pulse generator 55 controls emission of light of the LED 50 and outputs a pulse signal which indicates timing for causing an LED 51 to emit light, to the control unit 20A. The radiation dose calculation section 21A determines that an electrical signal which is synchronized with a pulse signal output from the pulse generator 55 is the detection result of the optical signal generated in the LED 50, among electrical signals output from the optical detector 15.

Accordingly, the radiation dose calculation section 21A can accurately discriminate the detection result of the optical signal generated in the LED 50 and the detection result of light generated in the scintillator 13 from each other, among the electrical signals output from the optical detector 15. Accordingly, the radiation dose calculation section 21A can accurately obtain the distribution of the wave heights X of the detection signals related to the optical signal generated in the LED 50, and the distribution of the wave heights Y of the detection signals related to light generated in the scintillator 13.

The present embodiment is configured as described above. The radiation dose calculation section 21A changes the determination threshold value $Q_{th}$ based on the optical signal from the LED 50. Therefore, it is possible to prevent detection accuracy of the neutron beam N further from being degraded, based on the optical signal from the LED 50.

The radiation dose calculation section 21A stores the distribution of the wave heights of the detection signals related to the optical signal from the LED 50 in advance. The radiation dose calculation section 21A can change the determination threshold value $Q_{th}$ in real time by comparing the distribution of the stored wave heights and the distribution of the wave heights of the detection signals actually measured by the optical detector 15 and related to the optical signal from the LED 50. Accordingly, even though the light guide 14 is deteriorated, it is possible to change the determination threshold value $Q_{th}$ in real time.

As the distribution of the stored wave heights is compared with the distribution of the wave heights of the detection signals actually measured by the optical detector 15 and related to the optical signal from the LED 50, it is possible to determine the presence and the absence of deterioration of the light guide 14A. Accordingly, the replacement time for the light guide 14A, and the like can be grasped.

As the LED 50 is installed in the cap 14b, it is possible to cause light generated in the LED 50 to be easily incident on the inside of the light guide 14A.

(Third Embodiment)

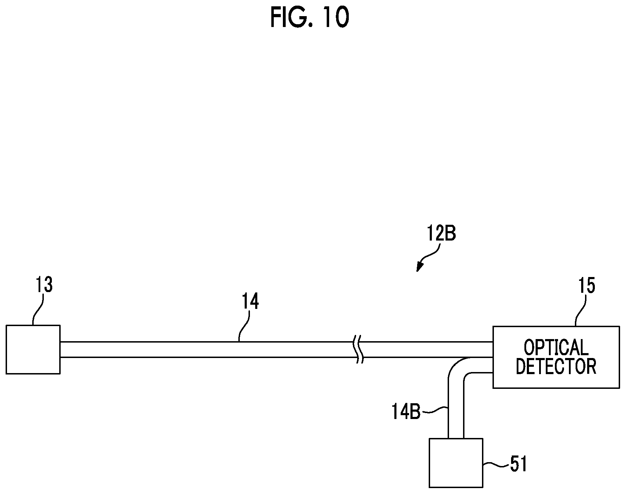

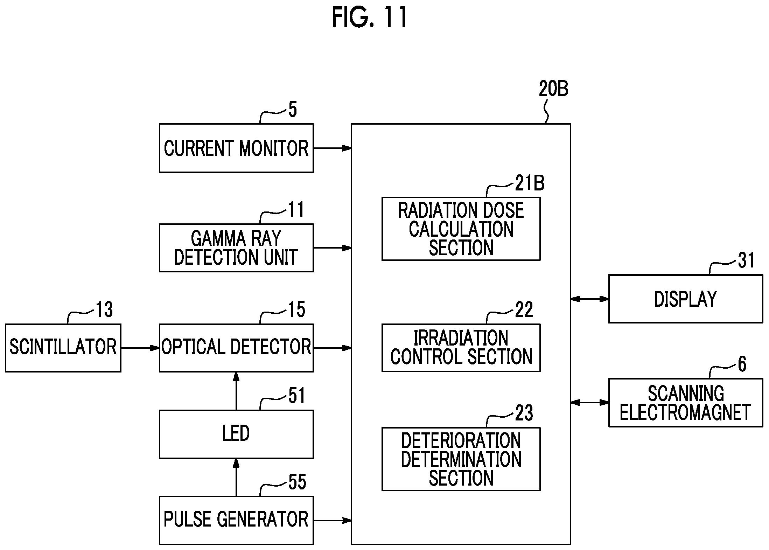

Subsequently, a third embodiment will be described. The main point of the present embodiment is that an LED light guide (a light generation section optical fiber) 14B and the LED 51 (refer to FIG. 10) are added to the first embodiment. In addition, the present embodiment uses a control unit 20B (refer to FIG. 11) of which the processing contents are different from that of the control unit 20 in the first embodiment. Hereinafter, descriptions will be given mainly regarding the points different from that in the first embodiment. In addition, the same reference numerals and signs are applied to the same elements as those in the first embodiment in the descriptions for the drawings, and overlapping descriptions will be omitted.

Here, when detecting light generated in the scintillator 13 by using the optical detector 15, there is a case where the quantity of light detected by the optical detector 15 varies. There is a case where the variation occurs due to instability of the optical detector 15, and there is a case where the variation occurs due to deterioration of the light guide 14 caused by radial rays.

In the related art, it has been difficult to determine whether the variation of the quantity of light detected by the optical detector 15 occurs due to instability of the optical detector 15 or occurs due to deterioration of the light guide 14 caused by radial rays. Therefore, an objective of the present embodiment is to determine whether the variation of the quantity of light detected by the optical detector 15 occurs due to instability of the optical detector 15 or occurs due to deterioration of the light guide 14.

As illustrated in FIG. 10, a neutron beam detector 12B includes the scintillator 13, the light guide 14, the optical detector 15, the LED 51, and the LED light guide 14B.

Emission of light in the LED 51 is controlled by the pulse generator 55 (refer to FIG. 11). The LED 51 is controlled by the pulse generator 55 and outputs a pulse-like optical signal. The optical signal generated in the LED 51 is transmitted to the optical detector 15 by the LED light guide 14B.

The optical detector 15 detects the optical signal generated in the LED 51, in addition to light which is generated in the scintillator 13. The optical detector 15 outputs an electrical signal (a detection signal) to the control unit 20B (refer to FIG. 11) when detecting light generated in the scintillator 13 or the optical signal generated in the LED 51.

As illustrated in FIG. 11, the control unit 20B includes a radiation dose calculation section 21B, the irradiation control section 22, and a deterioration determination section 23. The deterioration determination section 23 determines the presence and the absence of a problem in the optical detector 15 and deterioration of the light guide 14 based on the distribution of the wave heights of the detection signals detected by the optical detector 15 and related to light from the scintillator 13, and the distribution of the wave heights of the detection signals detected by the optical detector 15 and related to the optical signal from the LED 51.

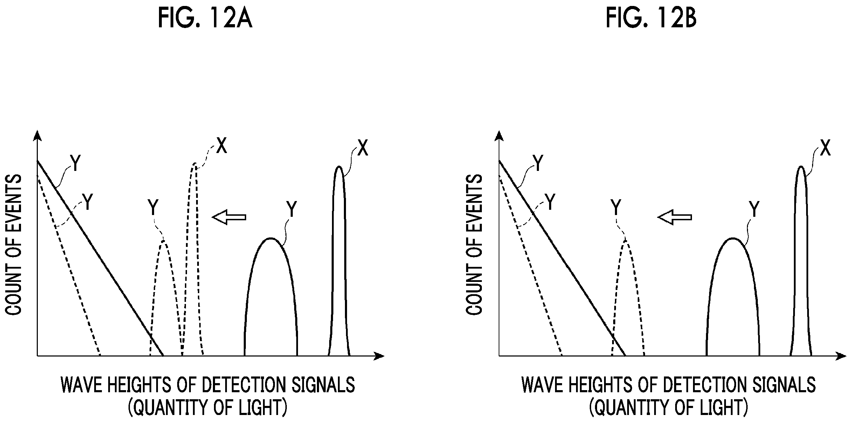

Here, FIG. 12(a) illustrates a graph of the distribution of the wave heights of the detection signals related to light received by the optical detector 15. As illustrated in FIG. 12, the graph of the distribution of the wave heights includes the distribution of the wave heights X of the detection signals related to the optical signal generated in the LED 51, and the distribution of the wave heights Y of the detection signals related to light generated in the scintillator 13.

For example, when a problem occurs in the optical detector 15 and the detection result of the optical detector 15 becomes unstable, the distribution of the wave heights X of the detection signals related to the optical signal generated in the LED 51, and the distribution of the wave heights Y of the detection signals related to light generated in the scintillator 13 are generally shifted in an increase direction or a decrease direction of the wave height as illustrated in FIG. 12 (a) . FIG. 12 (a) illustrates the distribution of the wave heights in a case of being shifted in the decrease direction of the wave heights, by using a dotted line.

Therefore, the deterioration determination section 23 determines that there is an occurrence of a problem in the optical detector 15 when the distribution of the wave heights X of the detection signals related to the optical signal generated in the LED 51, and the distribution of the wave heights Y of the detection signals related to light generated in the scintillator 13 are generally shifted. The deterioration determination section 23 may determine the presence and the absence of a problem in the optical detector 15 by comparing the distribution of the wave heights stored in advance and the distribution of the wave heights obtained based on the result which is actually measured by the optical detector 15. Otherwise, the deterioration determination section 23 may determine that there is an occurrence of a problem in the optical detector 15 when the distribution of the wave heights is shifted with the lapse of time.

In addition, for example, when there is an occurrence of deterioration of the light guide 14, only the distribution of the wave heights Y of the detection signals related to light generated in the scintillator 13 is shifted in the decrease direction of the wave height as illustrated in FIG. 12 (b) . FIG. 12(b) illustrates the distribution of the wave heights Y in a case of being shifted in the decrease direction of the wave heights, by using a dotted line. Since the optical signal from the LED 51 is not affected even though there is an occurrence of deterioration of the light guide 14, the distribution of the wave heights X does not vary.

Therefore, the deterioration determination section 23 determines that there is an occurrence of deterioration of the light guide 14 when only the distribution of the wave heights Y of the detection signals related to light generated in the scintillator 13 is shifted. The deterioration determination section 23 may determine the presence and the absence of deterioration of the light guide 14 by comparing the distribution of the wave heights stored in advance and the distribution of the wave heights obtained based on the result which is actually measured by the optical detector 15. Otherwise, the deterioration determination section 23 may determine that there is an occurrence of deterioration of the light guide 14 when the distribution of the wave heights Y is shifted with the lapse of time.

In addition, the pulse generator 55 controls emission of light of the LED 51 and outputs a pulse signal which indicates timing for causing the LED 51 to emit light, to the control unit 20B. The radiation dose calculation section 21B determines that an electrical signal which is synchronized with a pulse signal output from the pulse generator 55 is the detection result of the optical signal generated in the LED 51, among electrical signals output from the optical detector 15.

Accordingly, the radiation dose calculation section 21B can accurately discriminate the detection result of the optical signal generated in the LED 51 and the detection result of light generated in the scintillator 13 from each other, among the electrical signals output from the optical detector 15. Accordingly, the radiation dose calculation section 21B can accurately obtain the distribution of the wave heights X of the detection signals related to the optical signal generated in the LED 51, and the distribution of the wave heights Y of the detection signals related to light generated in the scintillator 13.

The present embodiment is configured as described above. The determination section determines the presence and the absence of a problem in the optical detector 15 and the presence and the absence of an occurrence of deterioration of the light guide 14 based on the distribution of the wave heights of the detection signals related to the optical signal from the LED 51, and the distribution of the wave heights of the detection signals related to light from the scintillator 13. Accordingly, it is possible to determine in real time whether variation is caused by a problem in the optical detector 15 or is caused due to deterioration of the light guide 14 when there is variation in the quantity of light detected by the optical detector 15. Since the cause of variation in the quantity of light detected by the optical detector 15 can be specified in real time, it is possible to correct instability of the optical detector 15 while detecting light.

The configuration described in the second embodiment in which the determination threshold value $Q_{th}$ is changed by installing the LED 50 at the tip end of the light guide 14A, and the configuration described in the third embodiment in which the presence and the absence of a problem in the optical detector 15 and the presence and the absence of an occurrence of deterioration of the light guide 14 are determined may be combined and used.

The present invention hereby is not limited to the first to third embodiments described above. Various changes can be made as follows without departing from the scope of the concept according to the present invention. For example, in the first to third embodiments, the neutron beam detection apparatus according to an aspect of the present invention is applied to the neutron capture therapy device 1. However, the usage of the neutron beam detection apparatus is not limited. For example, the neutron beam detection apparatus according to an aspect of the present invention may be applied to a monitor for monitoring the operational condition of a nuclear reactor. In addition, the neutron beam detection apparatus according to an aspect of the present invention may be used when measuring an accelerated neutron used in a physics experiment. In addition, the neutron beam detection apparatus according to an aspect of the present invention may be used in a neutron irradiation apparatus for non-destructive inspection.

Industrial Applicability

According to various aspects of the present invention, it is possible to provide a neutron beam detection apparatus and a neutron capture therapy device in which detection accuracy of a neutron beam can be prevented from being degraded even though an optical fiber is deteriorated and light transmittance thereof is degraded.

Reference Signs List

1 ... NEUTRON CAPTURE THERAPY DEVICE; 2 ... CYCLOTRON (ACCELERATOR) ; 7 ... TARGET; 12 ... NEUTRON BEAM DETECTOR; 13 ... SCINTILLATOR; 14, 14A ... LIGHT GUIDE (OPTICAL FIBER); 14B ... LED LIGHT GUIDE (LIGHT EMITTING SECTION OPTICAL FIBER); 15 ... OPTICAL DETECTOR; 20, 20A, 20B ... CONTROL UNIT; 21, 21A, 21B ... RADIATION DOSE CALCULATION SECTION; 23 ... DETERMINATION SECTION; 50, 51 ... LED (LIGHT EMITTING SECTION); N ... NEUTRON BEAM; AND M ... NEUTRON BEAM GENERATION SECTION.

**Claims**

1. A neutron beam detection apparatus for detecting a neutron beam (N), the apparatus comprising:

   a scintillator (13) configured to generate light when a radial ray is incident thereon;
   an optical fiber (14, 14A) configured to cause the light generated in the scintillator (13) to be transmitted therethrough; and
   a discrimination section configured to receive the light transmitted through the optical fiber (14, 14A) and to discriminate a detection signal generated by a gamma ray from a signal related to the neutron beam (N) when a wave height of the detection signal related to the received light exceeds a determination threshold value,

   wherein the discrimination section is adapted to adjust the determination threshold value in accordance with deterioration of the optical fiber (14, 14A).

2. The neutron beam detection apparatus according to Claim 1,
   wherein the discrimination section is adapted to adjust the determination threshold value based on distribution of the wave heights of the detection signals.

3. The neutron beam detection apparatus according to Claim 2,
   wherein the discrimination section is adapted to detect a peak generated by the neutron beam (N) in the distribution of the wave heights and to adjust the determination threshold value based on the peak.

4. The neutron beam detection apparatus according to Claim 3,
   wherein the discrimination section is adapted to set a value lowered by a predetermined value from the peak as the determination threshold value.

5. The neutron beam detection apparatus according to Claim 4,
   wherein the discrimination section is adapted to detect the peak generated by the neutron beam (N) by fitting a Gaussian function to the distribution of the wave heights, and to cause a value lowered by 1.5 $\sigma$ from the peak to be the determination threshold value.

6. The neutron beam detection apparatus according to Claim 1, further comprising:

a light generation section (50),
wherein the discrimination section is adapted to adjust the determination threshold value based on an optical signal from the light generation section (50).

7. The neutron beam detection apparatus according to Claim 6,
wherein the discrimination section is adapted to store the predetermined optical signal from the light generation section (50), to compare the stored predetermined optical signal with the actually measured optical signal which is detected via the optical fiber (14, 14A), and to adjust the determination threshold value based on a comparison result.

8. The neutron beam detection apparatus according to Claim 6 or 7,
wherein the light generation section (50) is installed at an end portion of the optical fiber (14, 14A) on a side on which light generated in the scintillator (13) is incident.

9. The neutron beam detection apparatus according to Claim 6, further comprising:

a determination section configured to determine which occurs between an occurrence of deterioration of the optical fiber (14, 14A) caused by the radial ray and an occurrence of a problem in the discrimination section, based on the optical signal from the light generation section (50).

10. The neutron beam detection apparatus according to Claim 9, further comprising:

a light generation section optical fiber (14B) configured to transmit the optical signal generated by the light generation section (50),
wherein the discrimination section is configured to also receive the optical signal transmitted through the light generation section optical fiber (14B), and
wherein the determination section is adapted to determine that there is an occurrence of a problem in the discrimination section when the wave height of the detection signal related to the light which is received by the discrimination section and is generated in the scintillator (13) and the wave height of the detection signal related to the optical signal which is output from the light generation section (50) are generally shifted in an increase direction or a decrease direction of the wave height, and to determine that there is an occurrence of deterioration of the optical fiber (14, 14A) caused by the radial ray when only the wave height of the detection signal related to the light which is generated in the scintillator (13) is shifted in the decrease direction of the wave height.

11. A neutron capture therapy device (1), comprising:

the neutron beam detection apparatus according to any one of Claims 1 to 10.


**Patentansprüche**

1. Neutronenstrahldetektionsvorrichtung zum Detektieren eines Neutronenstrahls (N), wobei die Vorrichtung Folgendes umfasst:

einen Szintillator (13), der konfiguriert ist, Licht zu erzeugen, wenn ein radialer Strahl darauf fällt;
eine Lichtleitfaser (14, 14A), die konfiguriert ist, zu bewirken, dass das in dem Szintillator (13) erzeugte Licht durch sie hindurch übertragen wird; und
einen Diskriminationsabschnitt, der konfiguriert ist, das durch die Lichtleitfaser (14, 14A) übertragene Licht zu empfangen, und ein durch einen Gamma-Strahl erzeugtes Detektionssignal von einem Signal bezüglich eines Neutronenstrahls (N) zu diskriminieren, wenn eine Wellenhöhe des Detektionssignals bezüglich des empfangenen Lichts einen Bestimmungsschwellenwert übersteigt,

wobei der Diskriminationsabschnitt dafür ausgelegt ist, den Bestimmungsschwellenwert in Übereinstimmung mit einer Verschlechterung der Lichtleitfaser (14, 14A) anzupassen.

2. Neutronenstrahldetektionsvorrichtung nach Anspruch 1,
wobei der Diskriminationsabschnitt dafür ausgelegt ist, den Bestimmungsschwellenwert basierend auf der Verteilung der Wellenhöhen der Detektionssignale anzupassen.

**3.** Neutronenstrahldetektionsvorrichtung nach Anspruch 2,
wobei der Diskriminationsabschnitt dafür ausgelegt ist, einen durch den Neutronenstrahl (N) erzeugten Spitzenwert in der Verteilung der Wellenhöhen zu detektieren, und den Bestimmungsschwellenwert basierend auf dem Spitzenwert anzupassen.

**4.** Neutronenstrahldetektionsvorrichtung nach Anspruch 3,
wobei der Diskriminationsabschnitt dafür ausgelegt ist, einen um einen vorbestimmten Wert gegenüber dem Spitzenwert verringerten Wert als den Bestimmungsschwellenwert einzustellen.

**5.** Neutronenstrahldetektionsvorrichtung nach Anspruch 4,
wobei der Diskriminationsabschnitt dafür ausgelegt ist, den von dem Neutronenstrahl (N) erzeugten Spitzenwert durch das Anpassen einer Gauß-Funktion an die Verteilung der Wellenhöhen zu detektieren, und zu bewirken, dass ein vom Spitzenwert um 1,5 σ verringerter Wert der Bestimmungsschwellenwert wird.

**6.** Neutronenstrahldetektionsvorrichtung nach Anspruch 1, die ferner Folgendes umfasst:

einen Lichterzeugungsabschnitt (50),
wobei der Diskriminationsabschnitt dafür ausgelegt ist, den Bestimmungsschwellenwert basierend auf einem optischen Signal von dem Lichterzeugungsabschnitt (50) anzupassen.

**7.** Neutronenstrahldetektionsvorrichtung nach Anspruch 6,
wobei der Diskriminationsabschnitt dafür ausgelegt ist, das vorbestimmte optische Signal von dem Lichterzeugungsabschnitt (50) zu speichern, das gespeicherte vorbestimmte optische Signal mit dem tatsächlich gemessenen optischen Signal, das über die Lichtleitfaser (14, 14A) detektiert worden ist, zu vergleichen, und den Bestimmungsschwellenwert basierend auf einem Ergebnis des Vergleichs anzupassen.

**8.** Neutronenstrahldetektionsvorrichtung nach Anspruch 6 oder 7,
wobei der Lichterzeugungsabschnitt (50) an einem Endbereich der Lichtleitfaser (14, 14A) an einer Seite, an der das in dem Szintillator (13) erzeugte Licht einfällt, installiert ist.

**9.** Neutronenstrahldetektionsvorrichtung nach Anspruch 6, die ferner Folgendes umfasst:

einen Bestimmungsabschnitt, der konfiguriert ist, basierend auf dem optischen Signal aus dem Lichterzeugungsabschnitt (50) zwischen einem Auftreten einer durch den radialen Strahl verursachten Verschlechterung der Lichtleitfaser (14, 14A) und einem Auftreten eines Problems in dem Diskriminationsabschnitt zu unterscheiden.

**10.** Neutronenstrahldetektionsvorrichtung nach Anspruch 9, die ferner Folgendes umfasst:

eine Lichtleitfaser (14B) des Lichterzeugungsabschnitts, die konfiguriert ist, das von dem Lichterzeugungsabschnitt (50) erzeugte optische Signal zu übertragen,
wobei der Diskriminationsabschnitt konfiguriert ist, auch das durch die Lichtleitfaser (14B) des Lichterzeugungsabschnitts übertragene optische Signal zu empfangen, und
wobei der Bestimmungsabschnitt dafür ausgelegt ist, zu bestimmen, dass in dem Diskriminationsabschnitt ein Problem aufgetreten ist, wenn die Wellenhöhe des Detektionssignals bezüglich des von dem Diskriminationsabschnitt empfangenen und in dem Szintillator (13) erzeugten Lichts und die Wellenhöhe des Detektionssignals bezüglich des von dem Lichterzeugungsabschnitt (50) ausgegebenen optischen Signals im Allgemeinen in einer Zunahmerichtung oder einer Abnahmerichtung der Wellenhöhe verschoben sind, und zu bestimmen, dass eine durch den radialen Strahl verursachte Verschlechterung der Lichtleitfaser (14, 14A)aufgetreten ist, wenn nur die Wellenhöhe des Detektionssignals bezüglich des in dem Szintillator (13) erzeugten Lichts in der Abnahmerichtung der Wellenhöhe verschoben ist.

**11.** Neutroneneinfang-Therapieeinrichtung (1), die Folgendes umfasst:

die Neutronenstrahldetektionsvorrichtung nach einem der Ansprüche 1 bis 10.

## EP 3 006 961 B1

**Revendications**

1. Un appareil de détection de faisceau de neutrons destiné à détecter un faisceau de neutrons (N), l'appareil comprenant :

   un scintillateur (13) configuré pour générer de la lumière lorsqu'un rayon radial arrive sur celui-ci ;
   une fibre optique (14, 14A) configurée pour faire que la lumière générée dans le scintillateur (13) soit transmise par celle-ci ; et
   une section de différenciation configurée pour recevoir la lumière transmise par la fibre optique (14, 14A) et pour faire la différence entre un signal de détection généré par un rayon gamma et un signal associé au faisceau de neutrons (N) lorsqu'une hauteur d'onde du signal de détection associé à la lumière reçue dépasse la valeur d'un seuil de détermination,

   dans lequel la section de différenciation est adaptée pour régler la valeur du seuil de détermination en fonction d'une dégradation de la fibre optique (14, 14A).

2. L'appareil de détection de faisceau de neutrons selon la revendication 1,
   dans lequel la section de différenciation est adaptée pour régler la valeur du seuil de détermination en fonction de la distribution des hauteurs d'onde des signaux de détection.

3. L'appareil de détection de faisceau de neutrons selon la revendication 2,
   dans lequel la section de différenciation est adaptée pour détecter un pic généré par le faisceau de neutrons (N) dans la distribution des hauteurs d'onde et pour régler la valeur du seuil de détermination en fonction du pic.

4. L'appareil de détection de faisceau de neutrons selon la revendication 3,
   dans lequel la section de différenciation est adaptée pour établir une valeur diminuée d'une valeur prédéterminée par rapport au pic comme valeur du seuil de détermination.

5. L'appareil de détection de faisceau de neutrons selon la revendication 4,
   dans lequel la section de différenciation est adaptée pour détecter le pic généré par le faisceau de neutrons (N) en ajustant une fonction gaussienne à la distribution des hauteurs d'onde, et pour faire qu'une valeur diminuée de $1,5\sigma$ par rapport au pic soit la valeur du seuil de détermination.

6. L'appareil de détection de faisceau de neutrons selon la revendication 1, comprenant en outre :

   une section de génération de lumière (50),
   dans lequel la section de différenciation est adaptée pour régler la valeur du seuil de détermination en fonction d'un signal optique émanant de la section de génération de lumière (50).

7. L'appareil de détection de faisceau de neutrons selon la revendication 6,
   dans lequel la section de différenciation est adaptée pour stocker le signal optique prédéterminé émanant de la section de génération de lumière (50), pour comparer le signal optique prédéterminé stocké avec le signal optique réellement mesuré qui est détecté via la fibre optique (14, 14A), et pour régler la valeur du seuil de détermination en fonction d'un résultat de la comparaison.

8. L'appareil de détection de faisceau de neutrons selon les revendications 6 ou 7,
   dans lequel la section de génération de lumière (50) est installée dans une partie d'extrémité de la fibre optique (14, 14A) sur un côté sur lequel arrive la lumière générée dans le scintillateur (13).

9. L'appareil de détection de faisceau de neutrons selon la revendication 6, comprenant en outre :

   une section de détermination configurée pour déterminer quelle est la situation, entre l'existence d'une dégradation de la fibre optique (14, 14A) provoquée par le rayon radial et l'existence d'une anomalie dans la section de différenciation, en fonction du signal optique émanant de la section de génération de lumière (50).

10. L'appareil de détection de faisceau de neutrons selon la revendication 9, comprenant en outre :

    une fibre optique de section de génération de lumière (14B) configurée pour transmettre le signal optique généré par la section de génération de lumière (50),

dans lequel la section de différenciation est configurée pour recevoir également le signal optique transmis par la fibre optique de la section de génération de lumière (14B), et

dans lequel la section de détermination est configurée pour déterminer qu'il existe une anomalie dans la section de différenciation lorsque la hauteur d'onde du signal de détection associé à la lumière qui est reçue par la section de différenciation et est générée dans le scintillateur (13) et la hauteur d'onde du signal de détection associé au signal optique qui est émis par la section de génération de lumière (50) sont généralement décalées dans une direction d'augmentation ou dans une direction de diminution de la hauteur d'onde, et pour déterminer qu'il existe une dégradation de la fibre optique (14, 14A) provoquée par le rayon radial lorsque seule la hauteur d'onde du signal de détection associé à la lumière qui est générée dans le scintillateur (13) est décalée dans la direction de diminution de la hauteur d'onde.

11. L'appareil de traitement par capture de neutrons, comprenant :

l'appareil de détection de faisceau de neutrons selon l'une quelconque des revendications 1 à 10.

EP 3 006 961 B1

# FIG. 1

# FIG. 2

## FIG. 3

# FIG. 4

# FIG. 5

| DETERMINATION THRESHOLD VALUE Qth ($\sigma$) | DETECTION EFFICIENCY (%) |
|---|---|
| 1 | 84.1% |
| 1.5 | 93% |
| 2 | 99.8% |

## FIG. 6

# FIG. 7

FIG. 8

FIG. 9

CURRENT MONITOR — 5

GAMMA RAY DETECTION UNIT — 11

LED — 50

SCINTILLATOR — 13

OPTICAL DETECTOR — 15

PULSE GENERATOR — 55

20A

RADIATION DOSE CALCULATION SECTION — 21A

IRRADIATION CONTROL SECTION — 22

DISPLAY — 31

SCANNING ELECTROMAGNET — 6

EP 3 006 961 B1

# FIG. 10

12B

13

14

15
OPTICAL DETECTOR

14B

51

# FIG. 11

CURRENT MONITOR ⌒5

GAMMA RAY DETECTION UNIT ⌒11

SCINTILLATOR ⌒13

OPTICAL DETECTOR ⌒15

LED ⌒51

PULSE GENERATOR ⌒55

20B

RADIATION DOSE CALCULATION SECTION ⌒21B

IRRADIATION CONTROL SECTION ⌒22

DETERIORATION DETERMINATION SECTION ⌒23

DISPLAY ⌒31

SCANNING ELECTROMAGNET ⌒6

EP 3 006 961 B1

# FIG. 12A

# FIG. 12B

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 61071381 A
- US 20090014662 A1